(19) Europäisches Patentamt
European Patent Office
Office européen des brevets

(11) Veröffentlichungsnummer: **0 001 102**
**A1**

(12) # EUROPÄISCHE PATENTANMELDUNG

(21) Anmeldenummer: **78100814.9**

(22) Anmeldetag: **04.09.78**

(51) Int. Cl.$^2$: **C 07 J 1/00**
**C 07 J 5/00**

(30) Priorität: **02.09.77 CH 10733/77**

(43) Veröffentlichungstag der Anmeldung:
**21.03.79 Patentblatt 79/6**

(84) Benannte Vertragsstaaten:
**BE CH DE FR GB NL**

(71) Anmelder: **F.Hoffmann-La Roche & Co. Aktiengesellschaft**
**Abt. VIII-Pt**
**CH-4002 Basel(CH)**

(72) Erfinder: **Fujiwara, Mitsuhiko**
**1059-7 Fueta**
**Kamakura-shi Kanagawa-ken(JP)**

(72) Erfinder: **Fujiwara, Akiko**
**1059-7 Fueta**
**Kamakura-shi Kanagawa-ken(JP)**

(72) Erfinder: **Miyamoto, Chikara**
**505 Maruma Building 250 Kuden-cho Totsuka-ku**
**Yokohama-shi Kanagawa-ken(JP)**

(74) Vertreter: **Grossner, Lutz, Dr. et al,**
**Grenzacher Strasse 124 Postfach 601**
**CH-4002 Basel(CH)**

(54) **Verfahren zur Herstellung von 7-Hydroxysteroiden.**

(57) Steroide der Formel

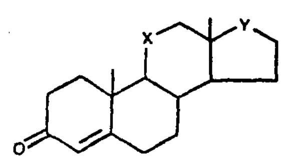

I

worin X Methylen, Hydroxymethylen oder Carbonyl und X einen Rest

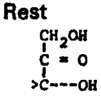 oder 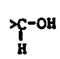 darstellt,

werden durch Mikroorganismen des Genus Mucor in 7$\alpha$-Stellung hydroxyliert. Die Verfahrensprodukte sind bekannte Verbindungen, die z.B. als Zwischenprodukte für die Synthese von Pharmazeutika von Wert sind.

EP 0 001 102 A1

0001102

RAN 4104/156

F. Hoffmann-La Roche & Co. Aktiengesellschaft, Basel/Schweiz

# Verfahren zur Herstellung von 7-Hydroxysteroiden

Die vorliegende Erfindung betrifft ein Verfahren zur Herstellung 7α-hydroxylierter Steroide durch Fermentierung 7-unsubstituierter Steroide der Pregnen- oder Androsten-Reihe mit Mikroorganismen des Genus Mucor.

Die mikrobiologische Herstellung von 7α-hydroxylierten Steroiden mittels Mikroorganismen der Genera Cephalosporium, Helminthosporium, Curvularia, Rhizopus, Aspergillus, Diplodia und Cunninghamella wurde bereits beschrieben. Die selektive Einführung einer Hydroxygruppe in 7α-Stellung von Steroiden der nachstehenden Formel I mittels Mikroorganismen des Genus Mucor wurde jedoch noch nicht beschrieben.

Das erfindungsgemässe Verfahren ist dadurch gekennzeichnet, dass man eine Verbindung der allgemeinen Formel

Grn/ 28.8.78

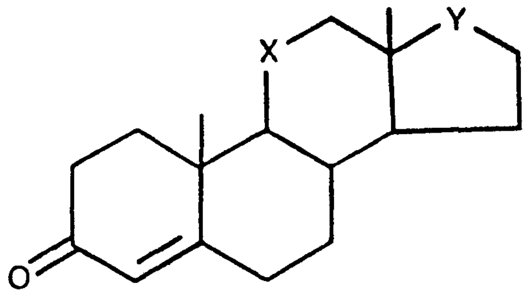

I

worin X $-CH_2-$, $-\overset{OH}{\overset{|}{C}}H-$ oder $-\overset{O}{\overset{\|}{C}}-$ und Y eine Gruppierung der Formeln 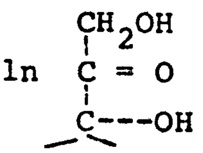 oder 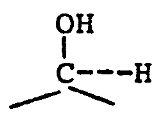 darstellt,

mit einem Mikroorganismus des Genus Mucor fermentiert. Das erfindungsgemässe Verfahren liefert Verbindungen der allgemeinen Formel

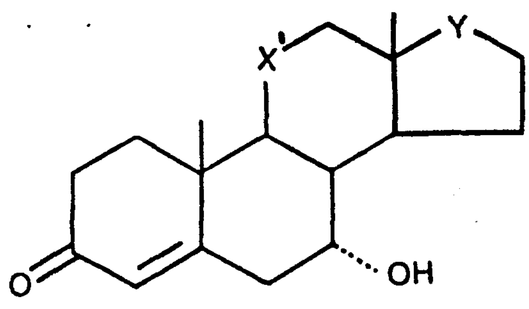

II

worin X' $-CH_2-$ oder $-\overset{O}{\overset{\|}{C}}-$ darstellt und Y die obige Bedeutung hat.

Erfindungsgemäss können alle Stämme des Genus Mucor, die zur 7α-Hydroxylierung von Steroiden der Formel I befähigt sind, verwendet werden. Bevorzugte Stämme sind Mucor spinescens IAM-6071 (FERM P-No. 4141), Mucor circinelloides ETH-2605 (FERM P-No. 4142), Mucor hiemalis IFO-8448 (FERM P-No. 4143), und IFO-8449 (FERM P-No. 4144), Mucor plumbeus CBS 295.63, Mucor hiemalis FERM-P No. 3800 und Varietäten davon.

Die Bezeichnung FERM P- bezieht sich auf Kulturen des Fermentation Research Institute, Chiba City, Japan (FRI); IAM bezeichnet das Institute of Applied Microbiology, University of Tokyo, IFO das Institute for Fermentation, Osaka, Japan; CBS das Centraal-bureau voor Schimmelcultures in Baarn, Niederlande; und ETH die Eidgenössische Technische Hochschule Zürich, Schweiz.

Die charakteristischen mykologischen Kennzeichen von Mucor hiemalis FERM-P No. 3800 sind die folgenden:

I. Wachstum

Kolonnien auf Malzextraktagar und Kartoffel-Dextrose-Agar wachsen sehr rasch, erreichen innerhalb von 3-5 Tagen bei 25$^{O}$C einen Durchmesser von 9 cm und bis zu 1,5 cm Höhe und sind hellgrau bis hell-olivgrau und flockig.

Bei 37$^{O}$C verläuft die Sprossung nur schwach und Wachstum ist makroskopisch nicht zu beobachten; gutes Wachstum und Sporulierung bei 5-30$^{O}$C, bei niedrigeren Temperaturen langsam. Die optimale Wachstumstemperatur ist 20-27$^{O}$C und das optimale pH 4,0-8,0.

II. Morphologie

Sporangiophoren haben einen Durchmesser bis zu 15 μ, sie sind leicht sympodisch verzweigt, mit oder ohne gelbliche Inhaltsstoffe. Verzweigungen sind oft leicht geschwollen und an der Base aufgerauht. Sporangia haben einen Durchmesser von 23,4-80,4 (-130) μ, sind kugelförmig, dunkelbraun bis fast schwarz mit zerfliessenden Wandungen.

Kolumellen haben eine Grösse von 19,2-41,8 x 13,4-36,7 μ, und sind überwiegend ellipsoid oder oboval, rundliche oder pyriforme kommen aber auch vor. Sporangiosporen sind in Grösse und Form variabel (5,0-16,7 x 3,3-8,4 μ), glattwandig und farblos, in Massen jedoch oliv bis olivbraun.

Zygosporen werden durch Kreuzen mit einem Minus-Stamm von Mucor hiemalis IFO-8449 auf Kartoffel-Dextrose-Agar bei 20$^{\circ}$C erhalten, der Durchmesser beträgt 36,7-80,0 μ, sie sind kugelförmig oder beinahe kugelförmig, braun bis fast schwarz und aufgerauht mit Stacheln. Zygosporenbildung wird bei Temperaturen von 25$^{\circ}$C oder höher nicht beobachtet.

Die morphologischen Charakteristiken des Stammes FERM-P No. 3800, kugelförmige Sporangien, Anwesenheit deutlicher Kolumellen und Fehlen von Apophyse, Stolon und Rhizoid, sind die Eigenschaften des Genus Mucor, die von J.A. von Arx in "The Genera of Fungi Sporulating in Pure Culture", 2nd edition, pp. 35-49, 1974 beschrieben sind. Dieses bekannte und grosse Genus wird in 7 Sektionen eingeteilt, von denen die Charakteristiken der Sektion Hiemalis recht enge morphologische Aehnlichkeiten mit denen des Stammes FERM-P No. 3800 in Bezug auf hellgraue bis hellolivgraue Kolonie, die Höhe von weniger als 2 cm, die sympodisch verzweigten Sporangiophoren, die bräunlich bis schwärzlich kurgelförmigen Sporangien mit einem Durchmesser von nicht über 100 μ zerfliessenden Wandlungen haben (siehe Zycha et al. in Mucorales, pp. 2-48, 1969).

Auf Grund der Klassifizierung der Sektion Hiemalis bei M.A.A. Schipper ("A Study on Variability in Mucor hiemalis and Relates Species" in Studies in Mycology, 1973, No. 4, pp. 1-40) gehört der Stamm FERM-P No. 3800 zu Mucor hiemals f. hiemalis, was durch die Bildung von Zygosporen mit einem Minusstamm von Mucor hiemalis IFO-8449 bestätigt wird.

Der vorliegende Stamm wird damit als Mucor hiemalis wehmer f. hiemalis identifiziert.

| Stamm | | CBS-No. | Hinterlegungsdatum |
|---|---|---|---|
| M. spinescens | IAM-6071 | | |
| M. circinelloides | ETH-2605 | | |
| M. hiemalis | IFO-8448 | | |
| M. hiemalis | IFO-8449 | | |
| M. hiemalis | FERM P-No.3800 | | |
| M. plumbeus | CBS 295.63 | | |

Die morpholischen Charakteristika von Mucor spinescens IAM-6071 und Mucor circinelloides ETH-2605 stimmen fast mit denen überein, die von M.A.A. Schipper ("A Study on Variability in Mucor spinescens and Mucor circinelloides and Relates Species" in "Studies in Mycology, No. 12, March 15, 1976"beschrieben werden.

Die Mikroorganismen können in Form der Kulturlösung, des Mycels oder in aufbereiteter Form verwendet werden. Die Kulturlösung kann durch Beimpfen eines geeigneten Mediums mit dem Mikroorganismus hergestellt werden. Das Kulturmedium kann Kohlenstoffquellen, Stickstoffquellen, anorganische Salze und andere für das Wachstum des Mikroorganismus geeignete Nährstoffe enthalten. Als Kohlenstoffquelle kommen beispielsweise Glucose, Sucrose, Dextrin, Mannose, Stärke, Lactose und Glycerin in Betracht; Stickstoffquellen sind beispielsweise stickstoffhaltige organische Substanzen wie Pepton, Fleischextrakt, Hefeextrakt, Maisquellwasser und Casein oder stickstoffhaltige anorganische Verbindungen wie Nitrate und anorganische Ammoniumsalze. Als anorganische Salze sind Phosphate oder Natrium-, Kalium-, Magnesium-, Mangan-, Eisen- und Kupfersalze zu erwähnen.

Die Züchtung des Mikroorganismus kann als Submerskultur, als Schüttelkultur oder als stationäre Kultur durchgeführt werden; vorzugsweise wird der Mikroorganismus unter aeroben Bedingungen kultiviert.

Das erfindungsgemässe Verfahren wird zweckmässig so durchgeführt, dass die zu hydroxylierende Verbindung der allgemeinen Formel I als Substrat zu der angezüchteten Kulturlösung gegeben wird. Die Konzentration des Substrats beträgt zweckmässig 0,1-20 g/l. Die erfindungsgemässe Hydroxylierung kann durch Fortsetzung der Kultivierung des Mikroorganismus unter den oben erwähnten Bedingungen durchgeführt werden. Die Reaktionszeit kann in Abhängigkeit von Spezies und Stamm des verwendeten Mikroorganismus, von der Zusammensetzung des Kulturmediums, vom verwendeten Substrat

und dessen Konzentration variiert werden. Im allgemeinen genügt eine Fermentationszeit von 1-10 Tagen. Die Reaktionstemperatur liegt allgemein zwischen 20 und 30°C, zweckmässig arbeitet man bei einem pH von 4-9.

Das Substrat kann der Kultur des Mikroorganismus auch während der Anzüchtung oder dem Kulturmedium vor der Sterilisation oder der Beimpfung zugesetzt werden.

Die erfindungsgemässe Hydroxylierung kann auch mit dem aus der Kulturlösung isolierten Mycel des Mikroorganismus oder mit einem aus der Kulturlösung oder dem Mycel in an sich bekannter Weise hergestellten Enzymextrakt durchgeführt werden. In diesem Falle kann die 7α-Hydroxylierung zweckmässig in Lösung, z.B. einer Pufferlösung, in physiologischer Salzlösung, in frischer Nährlösung oder in Wasser durchgeführt werden.

Das Ausgangsmaterial der Formel I kann als feines Pulver oder als Lösung in einem hydrophilen Lösungsmittel wie Aceton, Dimethylsulfoxyd, Methanol, Aethanol, Aethylenglykol, Propylenglykol oder Dioxan zugesetzt werden. Man kann auch einer wässrigen Suspension des Substrats ein oberflächenaktives Mittel oder ein Dispersionsmittel zusetzen oder das Substrat durch Behandlung mit Ultraschall emulgieren.

Beispiele von Ausgangsstoffen der Formel I sind 17α,21-Dihydroxy-4-pregnen-3,20-dion, 11β,17α,21-Trihydroxy-4-pregnen-3,20-dion, 17α,21-Dihydroxy-4-pregnen-3,11,20-trion und 17β-Hydroxy-4-androsten-3-on. Aus diesen Verbindungen werden die entsprechenden 7α-Hydroxyderivate erhalten, wobei bei Verwendung von 11β,17α,21-Trihydroxy4-pregnen-3,20-dion die 11-Hydroxygruppe gleichzeitig oxydiert wird.

Das Fermentationsprodukt kann aus dem Reaktionsgemisch in an sich bekannter Weise isoliert werden, beispielsweise durch Solventextraktion mit einem organischen Lösungsmittel,

das mit Wasser nicht mischbar ist, wie Chloroform, Methylenchlorid oder Aethylacetat; oder Chromtographie auf einem Träger wie Aluminiumoxyd, Silicagel oder Cellulose. Das Fermentationsprodukt kann auch durch Umkristallisation, z.B. aus Aethylacetat, Benzol oder Aceton gereinigt werden. Die erfindungsgemäss erhältlichen Verbindungen können als Zwischenprodukte zur synthetischen Herstellung von Hormonen, Chenodeoxycholsäure und anderen Pharmazeutika Verwendung finden.

Die folgenden Beispiele erläutern die Erfindung weiter.

Beispiel 1

Es wurde ein Kulturmedium mit 1% Maisquellwasser und 1% Glucose hergestellt und vor der Sterilisation auf pH 6,5 gestellt. 100 ml-Portionen dieses Mediums wurden auf zwanzig 500 ml-Erlenmeyer-Kolben verteilt, die Kolben mit Papierstopfen verschlossen und 15 Minuten bei 120°C sterilisiert. Nach dem Abkühlen wurden die Kolben mit dem Mycel einer 2 Wochen alten Agarkultur von Mucor hiemalis FERM P-3800 beimpft.

Die Kolben wurden dann auf einer Schüttelmaschine mit 180 Bewegungen/Min. bei 26,5°C geschüttelt. Nach 24 Stunden wurden in jedem Kolben 50 mg 17α,21-Dihydroxy-4-pregnen-3,20-dion in 1 ml Dimethylsulfoxyd gegeben. Die Inkubation wurde 69 Stunden fortgesetzt. Danach wurde die Kultur geerntet, filtriert und mit Wasser gewaschen. Das Filtrat und die Waschwässer wurden vereinigt und zweimal mit 2 l Aethylacetat extrahiert. Nach Trocknen über Natriumsulfat wurden die Extrakt unter vermindertem Druck auf ein kleines Volumen ölige Substanz eingeengt. Das Konzentrat wurde an einer Säule mit Kieselsäure (Mallincrodt) mit Chloroform-Aceton als Elutionsmittel chromatographiert. Mit einem Gemisch von Chloroform-Aceton (7:3) wurde das 7α,17α,21-Trihydroxy-4-pregnen-3,20-dion eluiert. Die zusammengehörenden Fraktionen wurden konzentriert und aus Aethylacetat umkristallisiert und lieferten 196 mg 7α,17α,21-Trihydroxy-4-pregnen-3,20-dion vom Schmelzpunkt 221,5-223,5°C.

Beispiel 2

In Analogie zu dem in Beispiel 1 beschriebenen Verfahren wurde je 1 Kolben mit a) Mucor spinescens IAM-6071, b) Mucor circinelloides ETH-2605, c) Mucor hiemalis IFO-8448, d) Mucor hiemalis IFO-8449 und e) Mucor plumbeus CBS 295.63 fermentiert. Die Ausbeuten an 7α,17α,21-Trihydroxy-4-pregnen-3,20-dion betrugen a) 10 mg, b) 4,5;

c) 7,5 mg; d) 15 mg; und e) 3 mg.

Beispiel 3

Ein Fermentationsmedium mit 1% Glucose und 1% Maisquellwasser wurde auf pH 6,5 gestellt. Dreissig 500 ml-Erlenmeyer-Kolben mit je 100 ml Medium wurden mit Mucor hiemalis FERM P-3800 beimpft und 2 Tage bei 26,5°C auf der Schüttelmaschine kultiviert. Danach wurden jedem Kolben 20 mg 11β,17α,21-Trihydroxy-4-pregnen-3,20-dion in 0,5 ml Dimethylsulfoxyd zugesetzt und die Inkubation wurde 5 Tage fortgesetzt. Die Kultur wurde filtriert und mit Wasser gewaschen. Filtrat und Waschwässer wurden mit Aethylacetat extrahiert und der Extrakt unter vermindertem Druck eingeengt. Chromatographie auf einer Silicagelsäule mit Chloroform-Aceton lieferte 58 mg 7α,17α,21-Trihydroxy-4-pregnen-3,11,20-trion, Schmelzpunkt 245,5-246,5°C; $\lambda_{max}$ in Methanol 238,5 nm (ε 10.729).

Beispiel 4

100 ml eines Mediums der gleichen Zusammensetzung wie in Beispiel 3 wurden in einem 500 ml-Erlenmeyer-Kolben mit Mucor hiemalis FERM P-3800 beimpft und 48 Stunden bei 26,5°C inkubiert. Danach wurden 10 mg 17α,21-Dihydroxy-4-pregnen-3,11,20-trion in 0,5 ml Dimethylsulfoxyd zugesetzt und die Kultur wurde 98 Stunden fermentiert. Man erhielt 1,1 mg 7α,17α,21-Trihydroxy-4-pregnen-3,11,20-trion.

Beispiel 5

100 ml des Mediums von Beispiel 1 wurden mit Mucor hiemalis FERM P-3800 beimpft und 22 Stunden bei 26,5°C geschüttelt. Die Kultur wurde dann mit 50 mg 17β-Hydroxy-4-androsten-3-on in 1 ml Dimethylsulfoxyd versetzt und 8 Tage inkubiert. Man erhielt aus dem Kulturfiltrat 4,8 mg 7α,17β-Dihydroxy-4-androsten-3-on vom Schmelzpunkt 191-193°C.

0001102

## Patentansprüche

1. Verfahren zur Herstellung von 7α-Hydroxy-Steroiden der Formel

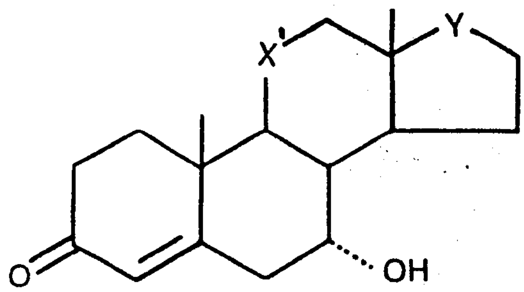

II.

worin X' $-CH_2-$ oder 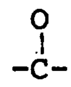 und Y eine Gruppierung der Formeln 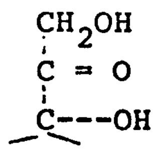 oder 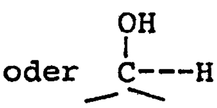 darstellt,

dadurch gekennzeichnet, dass man ein Steroid der Formel

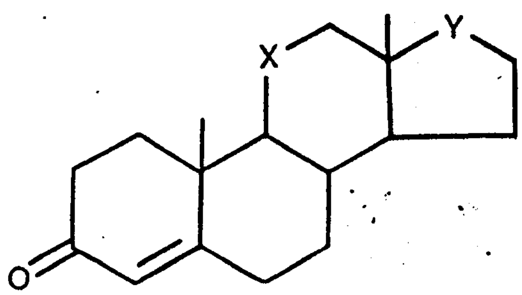

I

worin X $-CH_2-$, 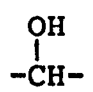 oder 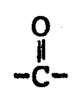 darstellt und Y die obige Bedeutung hat,

mit einem Mikroorganismus des Genus Mucor fermentiert.

2. Verfahren nach Anspruch 1, dadurch gekennzeichnet, dass man Mucor spinescens IAM-6071, Mucor circinelloides ETH-2605, Mucor hiemalis IFO-8448, Mucor hiemalis IFO-8449, Mucor plumbeus CBS 295.63 und Mucor hiemalis FRI FERM P-No. 3800 verwendet.

3. Verfahren nach Anspruch 1 oder 2, dadurch gekennzeichnet, dass man 17α,21-Dihydroxy-4-pregnen-3,20-dion, 11β,17α,21-Trihydroxy-4-pregnen-3,20-dion oder 17α,21-Dihydroxy-4-pregnen-3,11,20-trion fermentiert.

4. Verfahren nach Anspruch 1 oder 2, dadurch gekennzeichnet, dass man 17β-Hydroxy-4-androsten-3-on fermentiert.

***

Europäisches Patentamt

0001102

# EUROPÄISCHER RECHERCHENBERICHT

Nummer der Anmeldung

EP 78 10 0814

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | IL FARMACO EDIZIONE SCIENTIFICA vol. 28 (1973), Seiten 873-887.<br>* Seiten 873, 876, 877 * | 1 | C 07 J 1/00<br>C 07 J 5/00 |
| | IL FARMACO EDIZIONE SCIENTIFICA vol. 28 (1973), Seiten 1024-1029.<br>* Seiten 1024-1028 * | 1 | |
| | US - A - 2 960 513 (RICHARD W. THOMA)<br>* Beispiel 1 * | 1,3 | RECHERCHIERTE SACHGEBIETE (Int. Cl.²)<br>C 07 J 1/00<br>C 07 J 5/00 |
| | US - A - 2 962 512 (SEYMOUR BERNSTEIN)<br>* Spalten 1-4; Beispiel 1 * | 1,3 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 10-11-1978 | HENRY |